## Europäisches Patentamt

## European Patent Office

## Office européen des brevets

(19)

(11) Publication number: **0 144 964**
**B1**

(12) **EUROPEAN PATENT SPECIFICATION**

(45) Date of publication of patent specification: **17.02.88**

(51) Int. Cl.⁴: **C 07 C 126/08, C 05 C 9/00**

(21) Application number: **84114748.1**

(22) Date of filing: **04.12.84**

(54) **Process for concentrating an already concentrated urea solution to form a practically anhydrous melt.**

(30) Priority: **07.12.83 NL 8304211**

(43) Date of publication of application:
**19.06.85 Bulletin 85/25**

(45) Publication of the grant of the patent:
**17.02.88 Bulletin 88/07**

(84) Designated Contracting States:
**AT BE DE FR GB IT NL**

(56) References cited:
**FR-A-1 184 991**
**US-A-3 171 770**

(73) Proprietor: **STAMICARBON B.V.**
**Mijnweg 1**
**NL-6167 AC Geleen (NL)**

(72) Inventor: **Verweel, Cornelis**
**Boerhaavestraat 72**
**NL-6164 GT Geleen (NL)**

(74) Representative: **Hoogstraten, Willem Cornelis**
**Roeland et al**
**OCTROOIBUREAU DSM Postbus 9**
**NL-6160 MA Geleen (NL)**

Courier Press, Leamington Spa, England.

# Description

The invention relates to a process for concentrating an already concentrated urea solution to form a practically anhydrous melt.

In preparing urea from carbon dioxide and an excess of ammonia first a urea synthesis solution containing carbamate and free ammonia is formed in the synthesis section. The carbamate is decomposed into ammonia and carbon dioxide and the decomposition products are removed from the solution in a number of steps along with part of the ammonia and water present in the solution. In that process a urea solution in water is eventually obtained which contains about 70—75% (wt) urea and in which ammonia and carbon dioxide are still present. For fertilizing purposes and for use in the manufacture of resins this solution is not suitable. As a rule it must first be processed to form solid urea. In that process the approximately 25—30% (wt) water and the ammonia and carbon dioxide still present are removed by evaporation, or urea is allowed to crystallize out from the solution and the crystals are melted, upon which the resulting urea melt is converted into granular form. As in the exposure of urea solutions to high temperatures decomposition of urea and formation of biuret occur, which biuret is undersirable when using urea for fertilizing purposes as well as for the manufacture of resins, the evaporation of urea solutions is usually done under reduced pressure in order to avoid excessively high evaporation temperatures. Moreover, for economic reasons the evaporation is usually done in two or more steps, most of the water present being removed in the first (few) step(s) at a moderately reduced pressure, upon which the evaporation is continued in the final step under a far more strongly reduced pressure until a practically anhydrous urea melt is obtained containing less than 0.5% (wt) water.

If a urea solution or solid urea with a very low biuret content, for instance a biuret content lower than 0.3% (wt), is desired, urea is allowed to crystallize out from a moderately concentrated solution, for instance 70—85% (wt) urea solution, and the resulting crystals are melted. Depending on the amount of liquid adhering at the crystals, which liquid is supplied to the melting device along with the crystals, urea solutions are obtained in that process containing 1—2% (wt) water. In order to obtain non-caking urea grains, it is desirable for the water content of the resulting solution to be reduced to below 0.5% (wt) before further processing the solution.

From the US patent specification 3,171,770 a two-step evaporation process for urea solutions is known, in the first step of which the solutions are concentrated at a pressure higher than 200 mm Hg (0.267 bar) to 87—96% (wt) urea, upon which in the second step at a pressure lower than 100 mm Hg (0.133 bar), preferably between 50 and 80 mm Hg (0.067—0.107 bar), further evaporation takes place to a water content lower than 0.5% (wt) at temperatures lower than 145°C. In practice today mostly lower pressures are applied in the second evaporation step, for instance about 0.03 bar.

The water vapour released during the evaporation, which contains part of the ammonia and carbon dioxide still present in the urea solution to be evaporated, must be condensed with a view to the recovery of this ammonia and carbon dioxide, because by discharging this water vapour into the open air without such recovery losses of raw materials for the urea process would be involved and at the same time the environment would be poluted to an unacceptable degree. At a pressure of about 0.03 bar applied in the final evaporation step it is not possible, however, for the water vapour carried off from this step to be condensed by direct cooling with cooling water of, for instance, 25—40°C, because the vapour pressure of this cooling water is practically equal to or even higher than the pressure of the water vapour separated off. With a view to the said condensation of the water vapour by means of cooling water, the pressure of the water vapour separated off in the second evaporation step must be increased to a value definitely higher than the vapour pressure of the available cooling water. In practice this is usually done by allowing low-pressure steam to expand by means of an ejector, in which process, as a result of the expansion energy released, the desired reduced pressure is created in the evaporating space and the water vapour separated off from the solution is removed, which water vapour is compressed at the same time to the pressure of the expanded low-pressure steam. In view of the ever rising energy prices it is desirable, however, for the amount of steam required in the evaporating process to be reduced.

The object of the invention is to provide a concentration process for urea solutions in which, compared with the known processes, the amount of steam required is substantially reduced. This is achieved according to the invention in that in the final concentration step a pressure is applied higher than usual and subsequently in a separate step large part of the water still present in the partly concentrated urea solution is removed by expanding the solution in an expansion space and carrying off the water vapour released from the solution in the expansion. If the partly concentrated urea solution has been obtained by melting moist urea crystals, it will suffice as a rule to allow the resulting solution to expand without a further prior concentration step being necessary.

The invention therefore relates to a process for concentrating a partly concentrated urea solution, which process is characterized in that a urea solution obtained by evaporation at a pressure between 0.067 and 0.133 bar on a urea solution obtained by melting moist urea crystals, which contains at least 98% (wt) urea, is allowed to expand to a pressure between 0.013 and 0.067 bar and the released water vapour is removed.

The solution containing at least 98% (wt) urea can be obtained as known in the art, for instance by evaporation of dilute urea solutions in one or more steps, or by melting urea crystals still containing liquid from the crystallization process.

The pressure to which the already concentrated urea solution is allowed to expand may range from 0.067 to 0.013 bar. Pressures outside this range may be applied as well. However, if a urea solution containing at least 98% (wt) urea is allowed to expand to a pressure higher than 0.067 bar, the amount of water vapour separated off is extremely small, while, if the solution is allowed to expand to a pressure lower than 0.013 bar, uneconomically large amounts of low-pressure steam are required for maintaining the reduced pressure. Preference is given to allowing the urea solution to expand to a pressure between 0.020 and 0.033 bar, because in this range water vapour is properly separated off without excessive amounts of low-pressure steam being required for maintaining the reduced pressure. The water vapour separated off, which is compressed by the action of the ejector used for maintaining the reduced pressure in the expansion space, can be passed into, for instance, the vapour space of the final evaporating step or into the condenser forming part of this step.

It is also possible to condense, at least partly, the water vapour released in the expansion. The condensate obtained, which owing to the entrained liquid urea in the water vapour is in fact a diluted aqueous urea solution, can be recycled and concentrated together with the aqueous urea solution to be concentrated or be added to the already partially concentrated urea solution.

It has been found that in concentrating a urea solution in this manner to form a practically anhydrous melt the amount of low-pressure steam required is considerably smaller than the amount required in the evaporation according to the process known in the art. Moreover, as a result of the lower heat requirement, a smaller heating surface and a smaller vapour separator will suffice in the final evaporating step, so that this step may be smaller in design. At the same time, in condensing the steam-vapour mixture carried off, a substantialy smaller amount of process condensate is formed, so that the equipment and the amount of energy required for working it up is smaller. On the other hand, an ejector and space for expansion will be required for the separate step, but the costs involved are amply compensated by the resulting advantages.

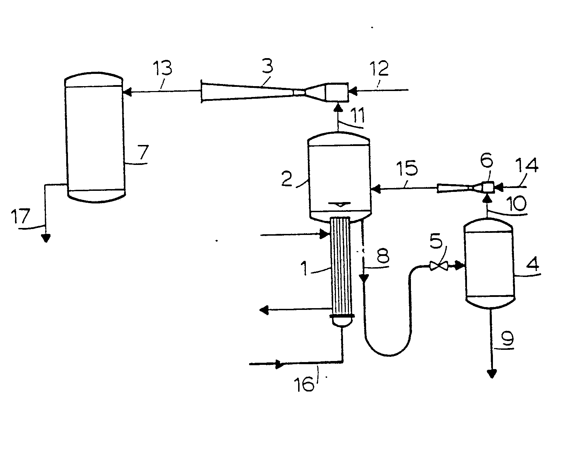

The invention will be elucidated by means of the figure and the examples without, however, being limited thereto.

In the figure, 1 and 2 represent respectively the heater and the vapour separator of an evaporator serving as the final evaporating step for a urea solution to be concentrated. 3 represents an ejector maintaining the reduced pressure in this evaporating step. 4 represents an expansion vessel, 5 an expansion valve and 6 an ejector maintaining in expansion vessel 4 a pressure lower than 0.067 bar, for instance 0.025 bar. A condenser is represented by 7.

The already fairly concentrated urea solution, for instance a 93—95% (wt) solution, is fed through 16 into heater 1 of the final evaporating step, which is heated, for instance, with low-pressure steam or a process stream suitable for that purpose. From the lower part of vapour space 2 the solution concentrated to more than 98% (wt) urea, for instance 99.2% (wt), is carried off through 8. This solution is passed via expansion valve 5 into expansion vessel 4, the water vapour formed in the expansion is sucked in through 10 by ejector 6, which is driven by low-pressure steam supplied through 14, and the resulting mixture of expanded low-pressure steam and released water vapour is brought to the pressure of the final evaporating step and is passed through 15 into vapour separator 2.

The resulting urea melt, which contains less than 0.5% (wt) water, for instance 0.3% (wt), is carried off through 9, for instance to a non-drawn shaping installation. The water vapour separated from the evaporated solution in vapour separator 2 and the mixture of steam and water vapour fed into this separator through 15 are sucked in through 11 by ejector 3, which is driven by low-pressure steam supplied through 12, while a usual vacuum of, for instance, 0.067—0.107 bar is created in the evaporator. The mixture of water vapour sucked in through 11 and low-pressure steam supplied through 12 is then brought to a pressure higher than 0.11 bar, so that condensation with cooling water of, for instance, about 30°C is possible. To this end the mixture flowing from ejector 3 is carried off through 13 to condenser 7. The condensate is carried off through 17. It is also possible for condenser 7 to be placed between vapour separator 2 and ejector 3.

Example I

With the process according to the invention a urea solution containing 5.5% (wt) water is evaporated to form a urea melt containing 0.3% (wt) water. Per hour 47,928 kg urea solution with a temperature of 130°C is passed into the evaporator, in which the pressure is kept at 0.077 bar by means of a steam-driven ejector 3. The urea solution carried off from the evaporator has a temperature of 140°C and still contains 0.78% (wt) water. The solution is passed into expansion vessel 4 in which a pressure of 0.025 bar is maintained using ejector 6. As a result of the lowering of the pressure, the temperature falls to 135°C. From the expansion vessel 45,133 kg urea melt, which still contains 0.3% (wt) water, is carried off. For maintaining the vacuum of 0.025 bar in the expansion space and compressing the water vapour to 0.077 bar using ejector 6, 560 kg low-pressure steam of 4 bar must be supplied hereto. For maintaining the vacuum of 0.077 bar in the evaporator and compressing the water vapour carried off to 0.113 bar 1960 kg low-pressure steam of 4 bar is required. Consequently, the total requirement amounts to 2520 kg low-pressure steam of 4 bar.

## Example II (comparative example) The same amount of urea solution of the same concentration and temperature as described in example 1 is evaporated to a water content of again 0.3% (wt) in an evaporator operated at 0.033 bar without applying the measure according to the invention. The vacuum is maintained by an ejector. From the evaporator 45,133 kg urea melt with a temperature of 140°C is carried off and 2813 kg of a vapour mixture which substantially consists of water vapour. For maintaining the vacuum 5,389 kg low-pressure steam of 4 bar is required.

## Claims

1. Process for concentrating a partly concentrated urea solution to form a practically anhydrous melt, characterized in that a urea solution obtained by evaporation at a pressure between 0.067 and 0.133 bar or a urea solution obtained by melting moist urea crystals, which contains at least 98 per cent by weight urea, is allowed to expand to a pressure between 0.013 and 0.067 bar and the released water vapour is removed.

2. Process according to claim 1, characterized in that the urea solution is allowed to expand to a pressure between 0.020 and 0.033 bar.

3. Process according to claim 1 or 2, characterized in that the water vapour released in the expansion is compressed and is passed into the condenser forming part of the final evaporating step.

4. Process according to claim 1 or 2, characterized in that the water vapour released in the expansion is compressed and is passed into the vapour separating space of the final evaporating step.

5. Process according to claim 1 or 2, characterized in that the water vapour released in the expansion is at least partially condensed and the condensate obtained is recycled and concentrated together with the aqueous urea solutions to be concentrated.

## Patentansprüche

1. Verfahren zum Konzentrieren einer teilweise konzentrierten Harnstofflösung, dadurch gekennzeichnet, daß eine durch Eindampfen bei einem Druck zwischen 0,067 und 0,133 bar erhaltene Harnstofflösung oder eine durch Schmelzen feuchter Harnstoffkristalle erhaltene Harnstofflösung, die wenigstens 98 Gew.-% Harnstoff enthält, auf einen Druck zwischen 0,013 und 0,067 bar expandieren gelassen und der freigesetzte Wasserdampf entfernt wird.

2. Verfahren nach Anspruch 1, dadurch gekennzeichnet, daß die Harnstofflösung auf einen Druck zwischen 0,020 und 0,033 bar expandieren gelassen wird.

3. Verfahren nach Anspruch 1 oder 2, dadurch gekennzeichnet, daß der bei der Expansion freigesetzte Wasserdampf verdichtet und in den einen Teil der Eindampfendstufe bildenden Kondensator eingeführt wird.

4. Verfahren nach Anspruch 1 oder 2, dadurch gekennzeichnet, daß der bei der Expansion freigesetzte Wasserdampf verdichtet und in den Dampfabscheideraum der Eindampfendstufe eingeführt wird.

5. Verfahren nach Anspruch 1 oder 2, dadurch gekennzeichnet, daß der bei der Expansion freigesetzte Wasserdampf wenigstens teilweise kondensiert und das Kondensat rückgeführt und zusammen mit den wässerigen, zu konzentrierenden Harnstofflösungen konzentriert wird.

## Revendications

1. Procédé pour concentrer une solution d'urée partiellement concentrée pour former une masse fondue pratiquement anhydre, caractérisé en ce qu'on décompresse jusqu'à une pression de 0,013 à 0,067 bar, une solution d'urée obtenue par évaporation à une pression de 0,067 à 0,133 bar ou une solution d'urée obtenue par fusion de cristaux d'urée humides, contenant au moins 98% en poids d'urée, et en ce qu'on élimine la vapeur d'eau dégagée.

2. Procédé suivant la revendication 1, caractérisé en ce que la solution d'urée est décompressée jusqu'à une pression de 0,020 à 0,033 bar.

3. Procédé suivant la revendication 1 ou 2, caractérisé en ce que la vapeur d'eau libérée lors de la décompression, est comprimée et introduite dans le condenseur de l'étape d'évaporation finale.

4. Procédé suivant la revendication 1 ou 2, caractérisé en ce que la vapeur d'eau libérée dans la décompression, est comprimée et introduite dans la chambre de séparation de vapeur de l'étape d'évaporation finale.

5. Procédé suivant la revendication 1 ou 2, caractérisé en ce que la vapeur d'eau libérée dans la décompression, est au moins partiellement condensée et en ce que le condensat obtenu est recyclé et concentré en combinaison avec les solutions aqueuses d'urée destinées à être concentrées.